# EUROPEAN PATENT SPECIFICATION

(11) **EP 1 723 433 B1**
(45) Date of publication and mention of the grant of the patent: **09.05.2012**
(21) Application number: 05708479.0
(22) Date of filing: 02.03.2005
(51) Int. Cl.: G01N 33/94, G01N 33/68, C12Q 1/00

(54) **DETECTION OF PRETERM LABOUR**
NACHWEIS VON FRÜHGEBURT
DETECTION D'UN ACCOUCHEMENT PREMATURE

(30) Priority: 08.03.2004 GB 0405133
(43) Date of publication of application: 22.11.2006
(73) Proprietor: University of Leicester, Leicester Leicestershire LE1 7RH (GB)
(72) Inventor: KONJE, Justin, Reproductive Sciences Section, Leicester, Leicestershire LE2 7LX (GB)
(74) Representative: Thomson, Craig Richard
(86) International application number: PCT/GB2005/050028
(87) International publication number: WO 2005/085873

(56) References cited:
- EP-A- 1 146 128
- MACCARRONE M ET AL: "A sensitive and specific radiochromatographic assay of fatty acid amide hydrolase activity" ANALYTICAL BIOCHEMISTRY, ACADEMIC PRESS INC. NEW YORK, US, vol. 267, 1999, pages 314-318, XP002154973 ISSN: 0003-2697
- PARIA B C ET AL: "Fatty-acid amide hydrolase is expressed in the mouse uterus and embryo durin the periimplantation period" BIOLOGY OF REPRODUCTION, vol. 60, May 1999 (1999-05), XP002154976 USA
- GIUFFRIDA, A. ET AL: "Quantification of bioactive acylethanolamides in rat plasma by electrospray mass spectrometry" ANALYTICAL BIOCHEMISTRY, vol. 280, 2000, pages 87-93, XP009047617
- HABAYEB, O.M.H. ET AL: "Endogenous cannabinoids: metabolism and their role in reproduction" LIFE SCIENCES, vol. 70, 2002, pages 1963-1977, XP009047619

## Description

### FIELD OF THE INVENTION

The present invention relates to a method for the detection of preterm labour in pregnant mammals. In particular, but not exclusively, the invention relates to such a method for use in humans.

### BACKGROUND OF THE INVENTION

Preterm labour is an important cause of morbidity and mortality among neonates. Accurate determination and detection of preterm labour can be critical in reducing the mortality rates, since rapid intervention can be beneficial. Accurate determination also assists in determining whether or not it is necessary to hospitalise the mother to allow for such interventions as are necessary. Further, inaccurate determination of preterm labour can lead to unnecessary hospitalisation, causing stress and discomfort to the mother, as well as using scarce healthcare resources.

Current markers used in diagnosing preterm labour include salivary estriol, foetal fibronectin, and insulin-like growth factor protein¹. The salivary estriol test detects elevated levels of estriol in a sample by means of ELISA (enzyme-linked immunosorbent assay), which are believed to be suggestive of preterm labour. Foetal fibronectin is usually detected from cervical swabs, and the absence of this protein is considered indicative that pregnancy will continue.

However, these methods are considered to be unreliable, and are not satisfactory. For example, the American College of Obstetricians and Gynecologists does not recommend the salivary estriol test as a screening tool for premature labour because it

Produces a high percentage of false positive results and could potentially add significant cost and unnecessary interventions to prenatal care. Similarly, the foetal fibronectin test is not recommended for routine screening, since while negative results are considered reliable for determining that labour has not begun, positive results are less reliable.

Additional methods for diagnosing preterm labour would therefore be desirable.

### SUMMARY OF THE INVENTION

The present invention relies on the identification of a novel marker for preterm labour. According to a first aspect of the present invention, there is provided a method for diagnosing preterm labour in a mammal, comprising detecting the level of an endocannabinoid in a sample from a subject mammal, and comparing the detected level with a reference level, an elevated level in the subject mammal being considered indicative of preterm labour.

The mammal may be a primate, and is preferably a human.

The detected level is preferably detected more than halfway through gestation. More preferably, the level is detected during late pregnancy, and conveniently at or near term. Late pregnancy in humans may refer to third trimester or later, preferably after the third trimester phase (above 35 weeks); most preferably term refers to 37 weeks or above.

The endocannabinoid is preferably anandamide (also knows as arachidonylethanolamide or AEA). In alternative embodiments of the Invention, the endocannabinoid may be 2-arachidonoylglycerol, or palmitoylethanolamide.

The reference level may be based on detected levels of the same subject mammal earlier in pregnancy, but is preferably based on typical non-term pregnancy levels for that species of mammal. The typical levels may be based on mid-term levels ; for example, where the method is to be used on humans, reference levels may be based on second or third trimester levels. The method may further comprise the step of taking a sample from the subject mammal for detection. The sample may be taken from blood, plasma, saliva, sputum, cervical or vaginal smears or swabs. The detection may be performed *in vitro.*

Detection of the level of the endocannabinoid may be performed in any suitable manner; for example, by means of HPLC, antibodies, receptors, binding molecules, and the like. In certain embodiments, the detection may involve the use of antibodies which bind the endocannabinoid; these antibodies may be labelled with fluorescent or radioactive markers to allow detection, or may themselves be subsequently detected with labelled secondary antibodies or enzymes. Alternatively the antibodies may be used to precipitate the endocannabinoid out of solution. The antibodies may be in solution, or affixed to a solid support. Receptors or binding molecules may be used to detect the endocannabinoid ; for example, the receptors CB1 and CB2 are known to bind to AEA, and may be used in detection of AEA.

In certain embodiments of the invention, the endocannabinoid levels may be detected indirectly. For example, it is believed that the enzyme fatty acid amide hydrolase (FAAH) degrades AEA, and that reduced levels of FAAH correlate with elevated levels of AEA. Detection of levels of FAAH may therefore be considered indicative of levels of AEA.

According to a further aspect of the present invention, there is provided a method for diagnosing preterm labour in a mammal, comprising detecting the level of the enzyme fatty acid amide hydrolase (FAAH) in a sample from a subject mammal, and comparing the detected level with a reference level, a reduced level in the subject mammal being considered indicative of preterm labour.

The detected level is preferably detected more than halfway through gestation. More preferably, the level is detected during late pregnancy, and conveniently at or near term. Late pregnancy in humans may refer to third trimester or later, preferably after the third trimester phase (above 35 weeks); most preferably term refers to 37 weeks or above.

Aspects of the invention also provide the use of an endocannabinoid in a method for diagnosing preterm labour in a mammal, the method comprising detecting the level of the endocannabinoid in a sample from a subject mammal, and comparing the detected level with a reference level, an elevated level in the subject mammal being considered indicative of preterm labour. The invention also relates to the use of an endocannabinoid in a method of diagnosis. Also provided is the use of FAAH in a method for diagnosing preterm labour in a mammal, the method comprising detecting the level of FAAH in a subject mammal, and comparing the detected level with a reference level, a reduced level in the subject mammal being considered indicative of preterm labour.

Although not a part of the present invention, also contemplated is a kit for use in diagnosis of preterm labour of a mammal, the kit comprising reagents for detection of levels of an endocannabinoid in a sample. The kit may further comprise instructions for use of the reagents. The reagents may comprise antibodies against the endocannabinoid, or may comprise receptors or other binding molecules to the endocannabinoid. The antibodies or binding molecules may be labelled, for example with a fluorescent or radioactive label; alternatively the kit may comprise additional reagents for detection of bound antibodies or binding molecules. The kit may further comprise means for taking a sample from a subject mammal; for example, swabs, syringes, and the like.

Although not part of the present invention, also contemplated is a method of treatment of preterm labour in a mammal, the method comprising reducing the levels of an endocannabinoid in a subject mammal experiencing preterm labour. The endocannabinoid is preferably AEA. The levels of the endocannabinoid may be reduced by administering antibodies or other binding molecules against AEA; or may be reduced by administering an enzyme which degrades the endocannabinoid, such as FAAH. Alternatively, the endocannabinoid may be 2- arachidonoylglycerol or palmitoylethanolamide. The use of an endocannabinoid in the preparation of a medicament for treatment of preterm labour in a mammal; and the use of FAAH in the preparation of a medicament for treatment of preterm labour in a mammal, is also contemplated (although this is not part of the present invention).

Although not part of the present invention, also contemplated is a method of inducing labour in a subject mammal, the method comprising elevating the effective levels of an endocannabinoid in a subject mammal.

The endocannabinoid is preferably AEA. The method may comprise the step of administering AEA itself, or may comprise administering an analogue thereto. Alternatively the levels of FAAH may be reduced, or a competitor to AEA may be administered to bind to FAAH, so reducing the effective levels thereof. Alternatively, the endocannabinoid may be 2-arachidonoylglycerol or palmitoylethanolamide.

### BRIEF DESCRIPTION OF THE FIGURES

These and other aspects of the invention will now be described by way of example only and with reference to the accompanying drawings, which show:
Figure 1. Plasma AEA levels in postmenopausal women, during the menstrual cycle, pregnancy, and in non-labour and labour women at term.
Figure 2: Relationship between plasma anandamide (AEA) levels and the duration of contractions at the time of sampling.
Figure 3: Relationship between plasma anandamide (AEA) levels and the interval from sampling to delivery.
Figure 4: Relationship between plasma anandamide (AEA) levels and cervical dilatation at the time of sampling.

### DETAILED DESCRIPTION OF THE DRAWINGS

### Introduction

Endogenous cannabinoids, endocannabinoids, are unsaturated fatty acid derivatives that act as ligands for the cannabinoid receptors. Arachidonylethanolamide or anandamide (AEA), was the first endocannabinoid isolated from brain tissue ². It is enzymatically released from cell membrane phospholipid precursors in response to depolarising agents, hormones and neurotransmitters ^{3 4}. The levels of AEA are considered to be controlled by its cellular uptake through an anandamide transporter ^{5, 6} and its subsequent enzymatic degradation by a membrane-bound fatty acid amide hydrolase (FAAH) ⁷⁻¹⁰ and other enzymes¹¹⁻¹³. AEA exerts its effects through interaction with plasma membrane cannabinoid receptors. Two subtypes of cannabinoid receptors, CB1 and CB2, belonging to the super family of G-protein coupled receptors ¹⁸ have been reported ¹⁴⁻¹⁷. They have been localised in different tissue types including the reproductive tract. The CB1 receptor is referred to as the central receptor, as it was first localised in the central nervous system ¹⁹ while the CB2 receptor is referred to as the peripheral receptor because it was first localised in the spleen and other sites ^{17,20,21}.

Several epidemiological and observational studies have been published on the adverse effects of exogenous cannabinoids present in marijuana on pregnancy in the human and animal models. These effects vary from early foetal loss, foetal growth restriction and prematurity ²²⁻²⁶. By contrast, there are relatively few reports on the role of endocannabinoids in pregnancy. In the mouse, it has been proposed that AEA plays an important role in the local regulation of uterine implantation ²⁷, since AEA was shown to be embryotoxic and arrests embryo development ²⁸. In the implantation site tissue AEA levels are only 25% of those in the non-implantation site ²⁹. This is considered to be due to local tissue levels of FAAH which is expressed at higher levels in the implantation site as compared to the non-implantation site ³⁰. More recent studies have suggested a further potential role for endocannabinoids (e.g. AEA) in myometrium since CB1R is expressed in the human myometrium and *in vitro* stimulation of this receptor by AEA results in relaxation of myometrial strips ³¹. CB1R and FAAH immunoreactivity have also recently been identified in the human placenta and foetal membranes ³², suggesting that the endocannabinoid system may also be important in these tissues during gestation.

Little information is available on the systemic levels of AEA during human pregnancy. However during early pregnancy blood AEA levels are inversely correlated with FAAH levels in peripheral blood mononuclear cells ³³ and since FAAH levels at 8 weeks gestation were lower in women who subsequently miscarried compared to those who progressed beyond the first trimester this suggested that low AEA levels are required for successful pregnancy progression³⁴. Plasma AEA levels have also been shown to be higher in patients who fail to achieve an ongoing pregnancy following IVF treatment and embryo transfer as compared to those who had successful ongoing pregnancies and was mirrored by lower levels of FAAH in peripheral blood mononuclear cells ³³. Whether these differences are influenced by or depend upon the various changes in the sex steroids that modulate implantation and maintain early pregnancy is uncertain. However the relationship between AEA levels and pregnancy establishment and in any other role is unclear since its levels have not been reported beyond the first trimester or indeed during the menstrual cycle. Such studies have been hampered by methodological difficulties in the past but the tools and techniques used for AEA assays have been and continue to be refined ³⁵.

Our objectives were, firstly, to refine and validate an established method to assay AEA in human plasma and then to use this method to determine, for the first time, plasma levels of AEA in the menstrual cycle and normal pregnancy.

### Subjects and methods

### Subjects:

All subjects gave a signed informed consent to take part in the study, which was approved by the local research ethics committee. For the purposes of determining the changes in the levels of AEA during pregnancy, we studied cross-sectionally 5 groups of women: first, second and third trimesters, term non-labouring and term labouring. The first trimester was defined as 6-11 postmenstrual weeks, the second 15-27 postmenstrual weeks and the third 28-35 postmenstrual weeks. Term was defined as 37-42 completed postmenstrual weeks.

The inclusion criteria for the pregnant women were accurately dated pregnancies from first trimesters ultrasound scans performed between 6 and 8 postmenstrual weeks, uncomplicated singleton pregnancies and no co-existing maternal or gestational diseases. None were on any medication or known to have taken recreational drugs. Only women in established labour (defined as cervical dilatation of at least 4cm and 3-4 regular uterine contractions every 10 minutes) were included in the term labouring group.

The non-pregnant women were divided into two groups: pre and post menopausal. The inclusion criteria for the pre-menopausal women included: aged between 22 and 40, body mass index (BMI) <27 kg/m², had not been on any hormonal contraception or therapy for at least three months, not on any drugs or suffering from any medical disorders. The samples were collected in two sub-groups based on the menstrual cycle (follicular: day 2-7 and luteal: day: 20-25) assessed from patient-reported last menstrual period date. The inclusion criteria for the post menopausal women included: a minimum of 2 years post menopause, BMI of <27 kg/m², aged between 55 and 60 years, not on hormone replacement therapy for the preceding three months and not on any drugs (e.g. glucocorticoids, antihypertensives, analgesics, recreational drugs) or suffering from any medical disorders.

### N-arachidonoylethanolamine (AEA) measurements:

Plasma AEA was measured by high performance liquid cluomatography-mass spectrometry (HPLC-MS) based on an isotope dilution method as reported previously ³⁶. Blood samples (4 ml) were collected in EDTA tubes on ice and added to polypropylene tubes containing 2 ml of Krebs-Tris-EDTA buffer. Samples were centrifuged at 1200 x g for 30 min at 22 °C to separate plasma from cells. After recovery, the plasma was spiked with 25.1 pmol of deuterium-labelled AEA (d₈-AEA; Cayman chemicals, Ann Arbor, USA) to estimate the efficiency of the lipid extraction procedure. Protein was precipitated by addition of ice-cold acetone followed by centrifugation at 1000 x g for 10 minutes at 22 °C. Lipid extraction was then performed on the supernatants by the addition of methanol: chloroform (1:2) (1:1 vol: vol). The chloroform layer was recovered and the samples were dried under a stream of nitrogen before reconstitution in methanol: chloroform (3:1). Duplicate injections of reconstituted sample (10 µl) were used for HPLC-MS analysis.

The HPLC-MS system consisted of a Waters 1525 binary Liquid Chromatography pump and a Waters 717 plus auto sampler fitted with a 100 µl injection loop. The HPLC was interfaced to a Quattro Ultima triple quadrupole mass spectrometer via a Z-spray ion source and controlled by MassLynx NT software v3.5 (Waters MS Technology, Manchester, UK). Reversed-phase chromatographic separations were performed on a Hypersil ODS C₁₈ column (4.6 x 100mm, 3 µm particle size; Phenomenex, Macclesfield, UK). The column temperature was maintained at 30 °C using a column temperature controller (Jones Chromatography, Hengoed, UK) and the samples at 4 °C in a refrigerated injection system. The mobile phase consisted of water (A) and methanol (B) delivered at 0.5 ml/min using a stepwise increase in methanol, thus: 25% A, 75% B for 2 min; 15% A, 85% B for 3 min; 5% A, 95% B for 20 min; 100% B for 5 min; a column re-equilibration step was then performed by changing the buffer conditions to 25% A, 75% B for 20 minutes. Under these conditions, the retention time for AEA and AEA-d₈ was 17.12 minutes and 16.98 minutes, respectively.

Electrospray ionisation was carried out in the positive mode using nitrogen as the nebulising gas. Capillary voltage was set at 3.5kV, cone voltage at 50V, source temperature 120 °C and desolvation temperature 350 °C. Mass spectrometry parameters for AEA-d₀ and AEA-d₈ analysis were established by infusion standards at 1 ng/µl using an infusion pump. Sodium adducts of the molecular ions [M+Na⁺; m/z=370.3 (do) and 378.3 (d₈)] were used for quantification in the selected ion recording mode. The measurements were expressed as the peak area ratio of the area under the curve for AEA-d₀: the area under the curve for AEA-d₈ and quantification of the amount of AEA in plasma samples spiked with the same amount of AEA-d₈ was made directly from a plot of peak area ratio versus known AEA-d₀ amounts (Cayman chemicals, Ann Arbor, USA) injected into the HPLC. Standard curves were linear up to 2 pmol AEA-d₀, with a lower limit of detection of 0.03125 pmol AEA-d₀ injected on the column. The intra- and inter-assay coefficients of variation, for the range of detection (0.03-2.1 pmol/column) were 6% and 9% respectively.

All the blood samples were processed within two hours of collection. Failure to process the sample within this time period led to degradation of AEA (data not shown).

### Statistical analyses:

Power analysis of published AEA data ³³ with α = 0.05 and β = 0.8 indicated that the minimum number of subjects required in each study group that would allow a significant change in plasma AEA concentrations to be observed was six. Therefore, we ensured that at least six subjects were included in each sample group (Table 1). Data are expressed as mean ± standard error of the mean (SEM) or standard deviation (SD) where appropriate for each test group and comparison between groups was performed using unpaired Student t-test with Welch's Correction for data with non-equivalent standard deviations and a p< 0.05 was considered statistically significant

### Results

### Subjects:

One hundred and two pregnant, menstruating and post-menopausal women were studied. There were 10 each in the first, second and third trimesters, 22 in term and non-labouring and 25 in term and labouring groups. Table I shows the details of these women. There were no significant differences in the mean ± SD ages of the women of reproductive age but the mean ± SD of the postmenopausal women was significantly greater than that of the non-pregnant menstruating group (57 ± 2.0 cf. 32.7 ± 6.1; P<0.05). The mean ± SD gestational age of the term non-labouring and labouring women was similar (39.3 ± 1.3 cf. 39.6 ± 1.3; P>0.05).

**Table I. Distribution, age and gestational age of the study groups (data are presented as means with ranges in parentheses).**

| **Study Group** | **Number** | **Age** | **Gestational age** |
|---|---|---|---|
| First trimester | 10 | 30.4 (25-36) | 9.1(6-11) |
| Second trimester | 10 | 28.1 (20-37) | 18.7 (14-26) |
| Third trimester | 10 | 26.3 (21-34) | 31.9 (28-35) |
| Term (not labouring) | 22 | 30.4 (22-37) | 39.3 (37-42) |
| Term (labouring) | 25 | 29.5 (18-40) | 39.5 (37-42) |
| Menstrual cycle (follicular) | 9 | 31.2 (23-39) | - |
| Menstrual cycle (luteal) | 8 | 34.2 (22-40) | - |
| Post menopausal | 8 | 57.7 (55-60) | - |

Seventeen of the 25 women in the term labouring group had a spontaneous vaginal delivery, 3 had a forceps delivery and 5 had operative deliveries (2 a vacuum extraction and 3 an emergency caesarean section). The mean ± SD cervical dilatation at the time of sampling was 6.04 ± 2.2 cm (range 4-10 cm) while the mean duration of uterine contractions before the blood sample collection was 5.1 ±2.2 hours (range 1.2-12 hours). The mean ± SD interval between sampling and delivery was 5.3 ± 4.3 hours (range 0.5-11.8 hours). Thirteen women had epidural analgesia in labour before sampling and the others (12) had only a combination of nitrous oxide and oxygen otherwise known as 'gas and air' in labour.

### N-arachidonoylethanolamine (AEA) levels:

The levels of AEA during pregnancy excluding the labouring women fell from 0.89 ± 0.14 nM in the first trimester to 0.44 ± 0.12 nM in the second and 0.44 ± 0.11 nM in the third trimesters (P=0.04) and thereafter rose to 0.68 ± 0.09 nM in the term non-labouring group (P=0.03). There was, however, a very dramatic change in the levels in the term labouring women, where they rose 3.7 times the values in term non-labouring women and 6 times those in the third trimester to 2.5 ± 0.22 nM. The differences between term labouring and the first, second and third trimesters and non-labouring levels were all statistically significant (P<0.0001).

In the menstruating women, the levels of AEA in the follicular phase (1.68 ± 10.16 nM) were significantly higher than those in the luteal phase (0.87 ± 0.19 nM); (P<0.0053). The levels in post-menopausal women (0.67 ± 0.01nM were significantly lower than those in the follicular phase (P=0.0002) but not significantly lower than those in the luteal phase.

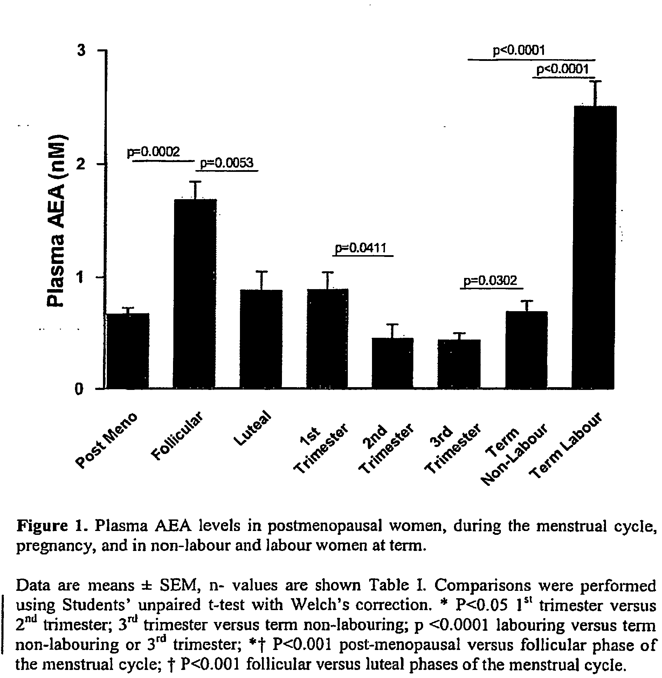

When the levels of AEA in the menstruating and postmenopausal women were compared to those during pregnancy, significant patterns emerged. These are shown in Figure 1. The post menopausal (0.67±0.01nM) and luteal phase levels (0.77±0.19 nM) were similar to those in the first trimester, higher (but not statistically, P>0.05) than the second and third trimesters and non-labouring term levels but significantly lower than the levels in the term labouring group (P<0.0002). The follicular phase levels were significantly higher (P<0.001) than the levels during pregnancy but significantly lower (P<0.005) than those in the labouring group.

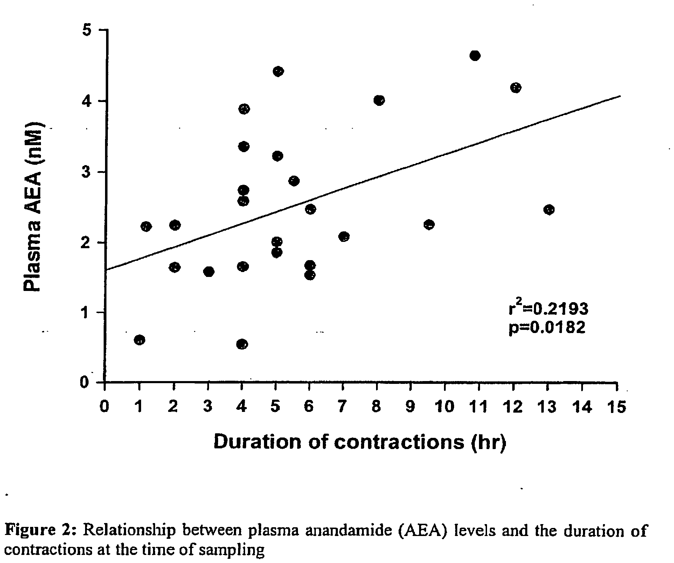

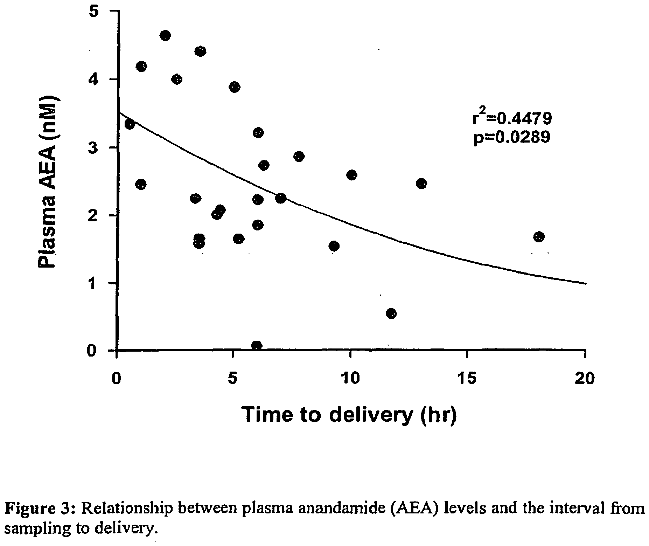

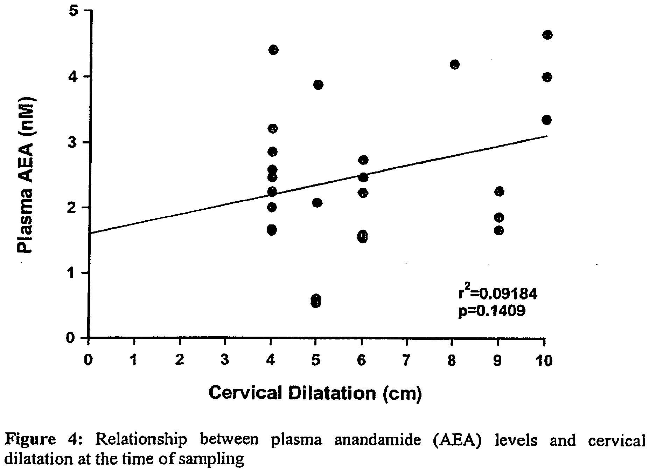

Figure 2 shows the relationship between plasma AEA levels and the duration of contractions up to the time of sampling. There was a direct linear relationship between AEA levels and this duration (r²= 0.2193, p<0.0182). The labouring women were further divided into three groups based on the total duration of labour (group I =0-5 hours, Group II=5.1-10 hours and group III=10.1-15 hours). The mean ± SEM levels of AEA in the three groups were respectively 2.1 ± 1.04 nM, 2.58 ± 0.95 and 3.8 ± 1.15 nM. Although there was a trend for AEA values to increase with increasing duration of labour, this did not achieve statistical significance (P>0.05). Figure 3 shows an inverse relationship between the levels of AEA at the time of sampling and the time from sampling to delivery. Figure 4 shows the relationship between the cervical dilatation at the time of sampling and plasma AEA levels; (r² =0.092, P=0.1). The relationship between plasma AEA and the interval between sampling and delivery is shown in Figure 4 (r²=2.113, P=0.02).

The levels in the 13 women who had an epidural analgesia before sampling were similar (2.24 ±0.32) to those in the 12 that had entonox 'gas and air' only (2.78 ± 0.30) prior to sampling and for the rest of their labour. None of the women received opiates.

### Discussion

Our results demonstrate remarkable changes in the levels of AEA during pregnancy and labour. Plasma AEA levels fell from the first to second and third trimesters with no change between the second and third trimesters. The levels rose at term prior to the onset of clinically apparent labour and rose further during labour to represent a 6-fold increase from third trimester levels.

The studies of Macarrone and colleagues ³³ demonstrated that, in IVF pregnancies, low plasma levels AEA were associated with pregnancies that progressed beyond the first trimester of pregnancy indicating that a successfully developing conceptus suppresses systemic levels of plasma AEA. That this suppression may be required for normal development of the foetus and pregnancy maintenance is supported by the association of high AEA levels with miscarriages³³. Endocannabinoids are also known to have adverse effects on successful pregnancy in mice (Yang et al, 1996; Schmid et al. 1997, Paria et al. 1999, Macarrone et a1 2000). High levels have been demonstrated to block the development of two-cell mouse embryos at the blastocyst stage and at blastocyst hatching (Yang et al, 1996), a mechanism, which may be associated with pregnancy failure or growth restriction of early onset. The requirement for an environment with low levels of AEA is supported by the effects of exogenous cannabinoids, which act upon the endocannabinoid receptors, and which are associated with miscarriages, foetal growth restriction and preterm labour ²²⁻²⁶.

Surprisingly in our study the levels detected during in the first trimester, which were similar to those reported in successful IVF pregnancies (Macarrone et al 2000), were similar to those in the luteal phase of the menstrual cycle. This implies that the low levels of AEA proposed to be required to support early pregnancy are already established in the luteal phase, enabling successful implantation, and that successful pregnancy represents a successful maintenance of these suppressed levels. The low levels in postmenopausal women and the high levels in the follicular phase, suggest that steroid hormones primarily regulate AEA levels, with oestradiol increasing the levels and progesterone suppressing them. The effect of progesterone could result from regulation of the degradation of peripheral AEA by peripheral blood mononuclear cells since the levels of FAAH in these cells, the principal enzyme involved in AEA degradation, are regulated by progesterone ^{37,38}. The induction of high AEA levels by oestradiol could be mediated by its effect upon endothelial cells since it has been reported that oestradiol increases the release of AEA from these cells into the circulation.³⁹

However these considerations do not account for the dramatic increase in the levels of AEA at term prior to the onset of clinically apparent labour and subsequently during labour when there are no changes in systemic steroid hormone levels. It is unlikely that the increase in AEA concentrations are due to pain because there was no correlation between AEA concentrations and the use of epidural analgesia during labour. Unless AEA production is uniquely stimulated by labour the previous considerations of steroid hormone control of its production and degradation could be explicable if, as has been proposed for the uterus (ref), that a 'functional progesterone withdrawal' occurs in peripheral blood mononuclear cells such that the induction of FAAH by progesterone fails allowing AEA levels to rise.

These observations beg the question as to the possible function of AEA in parturition and labour. Labour is associated with an increased local production of prostaglandins ⁴⁴ that act on all reproductive tissues and since AREA acts as a reservoir for arachidonic acid, the rise in AEA levels could be to provide a large reservoir of the precursor for prostaglandins production. Whether equivalent rises in AEA occur during preterm labour awaits further study. The cannabinoid receptors, CB1 and CB2, are present in the uterus (REF) and *in-vitro* stimulation of these receptors results in relaxation of myometrial strips (REF), which appears paradoxical given the rise in AEA associated with labour in this study. However it is possible that if the cannabinoid receptors are differentially expressed within the uterus then AEA action could be associated with relaxation of myometrial cells within the lower uterine segment during labour. This interpretation may be supported by our observation that AEA levels rose at term prior to the onset of clinically apparent uterine contractions and labour, and a period when development of the lower uterine segment and cervical dilation occur.

In conclusion these observations underline the potential role of endocannabinoids in human pregnancy maintenance and labour.

### References

1. Di Marzo V. 'Endocannabinoids' and other fatty acid derivatives with cannabimimetic properties: biochemistry and possible physiopathological relevance. Biochim Biophys Acta 1998; 1392:153-75.
2. Devane WA, Hanus L, Breuer A, et al. Isolation and structure of a brain constituent that binds to the cannabinoid receptor. Science 1992; 258:1946-9.
3. Piomelli D. The molecular logic of endocannabinoid signalling. Nat Rev Neurosci 2003; 4:873-84.
4. Habayeb OM, Bell SC, Konje JC. Endogenous cannabinoids: metabolism and their role in reproduction. Life Sci 2002; 70:1963-77.
5. Beltramo M, Stella N, Calignano A, Lin SY, Makriyannis A, Piomelli D. Functional role of high-affinity anandamide transport, as revealed by selective inhibition. Science 1997; 277:1094-7.
6. Hillard CJ, Edgemond WS, Jarrahian A, Campbell WB. Accumulation of N-arachidonoylethanolamine (anandamide) into cerebellar granule cells occurs via facilitated diffusion. J Neurochem 1997; 69:631-8.
7. Schmid PC, Zuzarte-Augustin ML, Schmid HH. Properties of rat liver N-acylethanolamine amidohydrolase. J Biol Chem 1985; 260:14145-9.
8. Cravatt BF, Giang DK, Mayfield SP, Boger DL, Lerner RA, Gilula NB. Molecular characterization of an enzyme that degrades neuromodulatory fatty-acid amides. Nature 1996; 384:83-7.
9. Ueda N, Kurahashi Y, Yamamoto S, Tokunaga T. Partial purification and characterization of the porcine brain enzyme hydrolyzing and synthesizing anandamide. J Biol Chem 1995; 270:23823-7.
10. Bracey MH, Hanson MA, Masuda KR, Stevens RC, Cravatt BF. Structural adaptations in a membrane enzyme that terminates endocannabinoid signaling. Science 2002; 298:1793-6.
11. Yu M, Ives D, Ramesha CS. Synthesis of prostaglandin E2 ethanolamide from anandamide by cyclooxygenase-2. J Biol Chem 1997; 272:21181-6.
12. Ueda N, Yamamoto K, Yamamoto S, et al. Lipoxygenase-catalyzed oxygenation of arachidonylethanolamide, a cannabinoid receptor agonist. Biochim Biophys Acta 1995; 1254:127-34.
13. Hampson AJ, Hill WA, Zan-Phillips M, et a1. Anandamide hydroxylation by brain lipoxygenase:metabolite structures and potencies at the cannabinoid receptor. Biochim Biophys Acta 1995; 1259:173-9.
14. Devane WA, Dysarz FA, 3rd, Johnson MR, Melvin LS, Howlett AC. Determination and characterization of a cannabinoid receptor in rat brain. Mol Pharmacol 1988; 34:605-13.
15. Matsuda LA, Lolait SJ, Brownstein MJ, Young AC, Bonner TI. Structure of a cannabinoid receptor and functional expression of the cloned cDNA. Nature 1990; 346:561-4.
16. Gerard CM, Mollereau C, Vassart G, Pannentier M. Molecular cloning of a human cannabinoid receptor which is also expressed in testis. Biochem J 1991; 279 (Pt 1):129-34.
17. Munro S, Thomas KL, Abu-Shaar M. Molecular characterization of a peripheral receptor for cannabinoids. Nature 1993; 365:61-5.
18. Howlett AC. Pharmacology of cannabinoid receptors. Annu Rev Pharmacol Toxicol 1995; 35:607-34.
19. Howlett AC. The cannabinoid receptors. Prostaglandins Other Lipid Mediat 2002; 68-69:619-31.
20. Kaminski NE, Abood ME, Kessler FK, Martin BR, Schatz AR. Identification of a functionally relevant cannabinoid receptor on mouse spleen cells that is involved in cannabinoid-mediated immune modulation. Mol Pharmacol 1992; 42:736-42.
21. Bouaboula M, Rinaldi M, Carayon P, et al. Cannabinoid-receptor expression in human leukocytes. Eur J Biochem 1993; 214:173-80.
22. Gibson GT, Baghurst PA, Colley DP. Maternal alcohol, tobacco and cannabis consumption and the outcome of pregnancy. Aust N Z J Obstet Gynaecol 1983; 23:15-9.
23. Zuckerman B, Frank DA, Hingson R, et al. Effects of maternal marijuana and cocaine use on foetal growth. N Engl J Med 1989; 320:762-8.
24. Frank DA, Bauchner H, Parker S, et al. Neonatal body proportionality and body composition after in utero exposure to cocaine and marijuana. J Pediatr 1990; 117:622-6.
25. McCance-Katz EF. The consequences of maternal substance abuse for the child exposed in utero. Psychosomatics 1991; 32:268-74.
26. Nair P, Rothblum S, Hebel R. Neonatal outcome in infants with evidence of foetal exposure to opiates, cocaine, and cannabinoids. Clin Pediatr (Phila) 1994; 33:280-5.
27. Paria BC, Song H, Wang X, et al. Dysregulated cannabinoid signaling disrupts uterine receptivity for embryo implantation. J Biol Chem 2001; 276:20523-8.
28. Paria BC, Das SK, Dey SK. The preimplantation mouse embryo is a target for cannabinoid ligand-receptor signaling. Proc Natl Acad Sci U S A 1995; 92:9460-4.
29. Schmid PC, Paria BC, Krebsbach RJ, Schmid HH, Dey SK. Changes in anandamide levels in mouse uterus are associated with uterine receptivity for embryo implantation. Proc Natl Acad Sci U S A 1997; 94:4188-92.
30. Paria BC, Deutsch DD, Dey SK. The uterus is a potential site for anandamide synthesis and hydrolysis: differential profiles of anandamide synthase and hydrolase activities in the mouse uterus during the periimplantation period. Mol Reprod Dev 1996; 45:183-92.
31. Dennedy MC, Houlihan AM, Friel AM, Smith T, Morrison JJ. Cannabinoids and the human uterus during pregnancy. Bjog 2003; 110:441.
32. Park B, Gibbons HM, Mitchell MD, Glassa M. Identification of the CB1 cannabinoid receptor and fatty acid amide hydrolase (FAAH) in the human placenta. Placenta 2003; 24:473-8.
33. Maccarrone M, Bisogno T, Valensise H, et al. Low fatty acid amide hydrolase and high anandamide levels are associated with failure to achieve an ongoing pregnancy after IVF and embryo transfer. Mol Hum Reprod 2002; 8:188-95.
34. Maccarrone M, Valensise H, Bari M, Lazzarin N, Romanini C, Finazzi-Agro A. Relation between decreased anandamide hydrolase concentrations in human lymphocytes and miscarriage. Lancet 2000; 355:1326-9.
35. Hillard CJ. Endocannabinoids and vascular function. J Pharmacol Exp Ther 2000; 294:27-32.
36. Giuffrida A, Rodriguez de Fonseca F, Piomelli D. Quantification of bioactive acylethanolamides in rat plasma by electrospray mass spectrometry. Anal Biochem 2000; 280:87-93.
37. Maccarrone M, Valensise H, Bari M, Lazzarin N, Romanini C, Finazzi-Agro A. Progesterone up-regulates anandamide hydrolase in human lymphocytes: role of cytokines and implications for fertility. J Immunol 2001; 166:7183-9.
38. Maccarrone M, Bari M, Di Rienzo M, Finazzi-Agro A, Rossi A. Progesterone activates fatty acid amide hydrolase (FAAH) promoter in human T lymphocytes through the transcription factor Ikaros. Evidence for a synergistic effect of leptin. J Biol Chem 2003; 278:32726-32.
39. Maccarrone M, Bari M, Battista N, Finazzi-Agro A. Estrogen stimulates arachidonoylethanolamide release from human endothelial cells and platelet activation. Blood 2002; 100:4040-8.
40. Smith R, Mesiano S, McGrath S. Hormone trajectories leading to human birth. Regul Pept 2002; 108:159-64.
41. Greene MF. Progesterone and preterm delivery-deja vu all over again. N Engl J Med 2003; 348:2453-5.
42. Bethin KE, Nagai Y, Sladek R, et al. Microarray analysis of uterine gene expression in mouse and human pregnancy. Mol Endocrinol 2003; 17:1454-69.
43. Wenger T, Fragkakis G, Giannikou P, Yiannikakis N. The effects of prenatally administered endogenous cannabinoid on rat offspring. Pharmacol Biochem Behav 1997; 58:537-44.
44. Challis JR, Sloboda DM, Alfaidy N, et al. Prostaglandins and mechanisms of preterm birth. Reproduction 2002; 124:1-17.

## Claims

1. A method for diagnosing preterm labour in a mammal, comprising detecting the level of an endocannabinoid in a sample from a subject mammal, and comparing the detected level with a reference level, an elevated level in the subject mammal being considered indicative of preterm labour.

2. The method of claim 1, wherein the mammal is a human.

3. The method of claim 1 or claim 2, wherein the endocannabinoid is selected from the group comprising 2-arachidonoylglycerol, palmitoylethanolamide, and anandamide.

4. The method of claim 3, wherein the endocannabinoid is anandamide (AEA).

5. The method of any preceding claim, wherein the reference level is based on typical non-term pregnancy levels for that species of mammal.

6. The method of claim 5, wherein the reference level is based on mid-term levels.

7. The method of claim 1, wherein the sample is plasma.

8. The method of any preceding claim, wherein the detection is performed in vitro.

9. The method of any preceding claim, wherein the detection is performed by means of antibodies to the endocannabinoid.

10. The method of any preceding claim, wherein the detection step comprises detection of the levels of fatty acid amide hydrolase (FAAH) in the subject mammal.

11. A method for diagnosing preterm labour in a mammal, comprising detecting the level of the enzyme fatty acid amide hydrolase (FAAH) in a sample from a subject mammal, and comparing the detected level with a reference level, a reduced level in the subject mammal being considered indicative of preterm labour.

12. The method of any preceding claim wherein the detected level is detected during late pregnancy.

13. The method of claim 12 wherein the detected level is detected at or near term.

14. The method of any preceding claim, when carried out on a human, wherein the detected level is detected during the third trimester or later.

15. The method of claim 14, wherein the detected level is detected at 35 weeks or later.

16. The use of an endocannabinoid in a method for diagnosing preterm labour in a mammal, the method comprising detecting the level of the endocannabinoid in a sample from a subject mammal, and comparing the detected level with a reference level, an elevated level in the subject mammal being considered indicative of preterm labour.

## Patentansprüche

1. Ein Verfahren zum Diagnostizieren von Frühwehen bei einem Säugetier, das das Ermitteln des Pegels eines Endocannabinoids in einer Probe von einem Säugetierindividuum und das Vergleichen des ermittelten Pegels mit einem Referenzpegel beinhaltet, wobei ein erhöhter Pegel in dem Säugetierindividuum als Frühwehen anzeigend angesehen wird.

2. Verfahren gemäß Anspruch 1, wobei das Säugetier ein Mensch ist.

3. Verfahren gemäß Anspruch 1 oder Anspruch 2, wobei das Endocannabinoid aus der Gruppe ausgewählt ist, die 2-Arachidonylglycerol, Palmitoylethanolamid und Anandamid beinhaltet.

4. Verfahren gemäß Anspruch 3, wobei das Endocannabinoid Anandamid (AEA) ist.

5. Verfahren gemäß einem der vorhergehenden Ansprüche, wobei der Referenzpegel auf typischen nichtterminalen Schwangerschafts-/Trächtigkeitspegeln für diese Art von Säugetier basiert.

6. Verfahren gemäß Anspruch 5, wobei der Referenzpegel auf Pegeln der mittleren Schwangerschaft/Trächtigkeit basiert.

7. Verfahren gemäß Anspruch 1, wobei die Probe Plasma ist.

8. Verfahren gemäß einem der vorhergehenden Ansprüche, wobei die Ermittlung in vitro durchgeführt wird.

9. Verfahren gemäß einem der vorhergehenden Ansprüche, wobei die Ermittlung mittels Antikörpern gegen das Endocannabinoid durchgeführt wird.

10. Verfahren gemäß einem der vorhergehenden Ansprüche, wobei der Ermittlungsschritt die Ermittlung der Pegel von Fettsäureamidhydrolase (FAAH) in dem Säugetierindividuum beinhaltet.

11. Ein Verfahren zum Diagnostizieren von Frühwehen bei einem Säugetier, das das Ermitteln des Pegels des Enzyms Fettsäureamidhydrolase (FAAH) in einer Probe von einem Säugetierindividuum und das Vergleichen des ermittelten Pegels mit einem Referenzpegel beinhaltet, wobei ein reduzierter Pegel in dem Säugetierindividuum als Frühwehen anzeigend angesehen wird.

12. Verfahren gemäß einem der vorhergehenden Ansprüche, wobei der ermittelte Pegel während der späten Schwangerschaft/Trächtigkeit ermittelt wird.

13. Verfahren gemäß Anspruch 12, wobei der ermittelte Pegel an oder nahe dem errechneten Geburtstermin ermittelt wird.

14. Verfahren gemäß einem der vorhergehenden Ansprüche, wenn bei einem Menschen ausgeführt, wobei der ermittelte Pegel während des dritten Trimesters oder später ermittelt wird.

15. Verfahren gemäß Anspruch 14, wobei der ermittelte Pegel zur 35. Woche oder später ermittelt wird.

16. Verwendung eines Endocannabinoids bei einem Verfahren zum Diagnostizieren von Frühwehen bei einem Säugetier, wobei das Verfahren das Ermitteln des Pegels des Endocannabinoids in einer Probe von einem Säugetierindividuum und das Vergleichen des ermittelten Pegels mit einem Referenzpegel beinhaltet, wobei ein erhöhter Pegel in dem Säugetierindividuum als Frühwehen anzeigend angesehen wird.

## Revendications

1. Une méthode pour diagnostiquer un travail avant terme chez un mammifère, comprenant la détection du niveau d'un endocannabinoïde dans un échantillon provenant d'un sujet mammifère, et la comparaison du niveau détecté avec un niveau de référence, un niveau élevé chez le sujet mammifère étant considéré comme indicateur de travail avant terme.

2. La méthode de la revendication 1, dans laquelle le mammifère est un humain.

3. La méthode de la revendication 1 ou de la revendication 2, dans laquelle l'endocannabinoïde est sélectionné dans le groupe comprenant le 2-arachidonoylglycérol, le palmitoyléthanolamide, et l'anandamide.

4. La méthode de la revendication 3, dans laquelle l'endocannabinoïde est l'anandamide (AEA).

5. La méthode de n'importe quelle revendication précédente, dans laquelle le niveau de référence est basé sur des niveaux typiques pour grossesse non à terme pour cette espèce de mammifère.

6. La méthode de la revendication 5, dans laquelle le niveau de référence est basé sur des niveaux à mi-terme.

7. La méthode de la revendication 1, dans laquelle l'échantillon est du plasma.

8. La méthode de n'importe quelle revendication précédente, dans laquelle la détection est effectuée in vitro.

9. La méthode de n'importe quelle revendication précédente, dans laquelle la détection est effectuée au moyen d'anticorps à l'endocannabinoïde.

10. La méthode de n'importe quelle revendication précédente, dans laquelle l'étape de détection comprend la détection des niveaux d'hydrolase d'amide d'acide gras (FAAH) chez le sujet mammifère.

11. Une méthode pour diagnostiquer un travail avant terme chez un mammifère, comprenant la détection du niveau de l'enzyme hydrolase d'amide d'acide gras (FAAH) dans un échantillon provenant d'un sujet mammifère, et la comparaison du niveau détecté avec un niveau de référence, un niveau réduit chez le sujet mammifère étant considéré comme indicateur de travail avant terme.

12. La méthode de n'importe quelle revendication précédente dans laquelle le niveau détecté est détecté en fin de grossesse.

13. La méthode de la revendication 12 dans laquelle le niveau détecté est détecté à terme ou près du terme.

14. La méthode de n'importe quelle revendication précédente, lorsque réalisée sur un humain, dans laquelle le niveau détecté est détecté au cours du troisième trimestre ou plus tard.

15. La méthode de la revendication 14, dans laquelle le niveau détecté est détecté à la 35^{ème} semaine ou plus tard.

16. L'utilisation d'un endocannabinoïde dans une méthode pour diagnostiquer un travail avant terme chez un mammifère, la méthode comprenant la détection du niveau de l'endocannabinoïde dans un échantillon provenant d'un sujet mammifère, et la comparaison du niveau détecté avec un niveau de référence, un niveau élevé chez le sujet mammifère étant considéré comme indicateur de travail avant terme.
